# EUROPÄISCHE PATENTSCHRIFT

(11) **EP 0 842 159 B1**
(45) Veröffentlichungstag und Bekanntmachung des Hinweises auf die Patenterteilung: **25.10.2000**
(21) Anmeldenummer: 96928367.0
(22) Anmeldetag: 22.07.1996
(51) Int. Cl.: C07D 251/16, C07D 239/46

(54) **VERFAHREN ZUR HERSTELLUNG VON HERBIZIDEN SULFONYLHARNSTOFFEN UND VON N-(PYRIMIDINYL- ODER -TRIAZINYL)-CARBAMATEN ALS ZWISCHENPRODUKTE**
METHOD OF PREPARARING SULPHONYL UREAS WITH HERBICIDAL PROPERTIES AND N-PYRIMIDINYL OR N-TRIAZINYL CARBAMATES AS INTERMEDIATES IN THE REACTION
PROCEDE DE PREPARATION DE SULFONYLUREES HERBICIDES ET DE N-(PYRIMIDINYL OU TRIAZINYL)-CARBAMATES COMME PRODUITS INTERMEDIAIRES

(30) Priorität: 02.08.1995 DE 19528303
(43) Veröffentlichungstag der Anmeldung: 20.05.1998
(73) Patentinhaber: Aventis CropScience GmbH, 65929 Frankfurt am Main (DE)
(72) Erfinder: SCHNABEL, Gerhard, D-63868 Grosswallstadt (DE); KNORR, Harald, D-60529 Frankfurt am Main (DE); MINN, Klemens, D-65795 Hattersheim (DE); VERMEHREN, Jan, D-65510 Idstein (DE)
(86) Internationale Anmeldenummer: EP9603219
(87) Internationale Veröffentlichungsnummer: WO9705121

(56) Entgegenhaltungen:
- EP-A- 0 562 576

## Beschreibung

Die Erfindung betrifft ein Verfahren zur Herstellung von Carbamaten, die sich zur Herstellung von herbizidwirksamen Sulfonylharnstoffen der Formel (I) eignen,

A-SO₂-NH-CO-NR-B (I)

worin
- A: einen organischen Rest, der gegebenenfalls Heteroatome enthält, beispielsweise einen aliphatischen, aromatischen oder heteroaromatischen Rest, der unsubstituiert oder substituiert ist und der direkt oder über eine divalente Gruppe mit Heteroatomen an der SO₂-Gruppe in Formel (I) gebunden ist,
- B: einen Pyrimidin-2-yl- oder 1,3,5-Triazin-2-yl-rest, der unsubstituiert oder substituiert ist, und
- R: Wasserstoff, C₁-C₅-Alkyl, C₁-C₅-Haloalkyl, C₁-C₅-Alkoxy oder C₁-C₅-Haloalkoxy
bedeuten.

Die Verbindungen der Formel (I) sind in der Regel bekannt und können als Pflanzenschutzmittel mit herbizider Wirkung eingesetzt werden; vgl. z.B. WO 92/00304, DE-A1-4236902, WO 89/10921, EP-A1-301784, EP-A1-187489.

Die bekannten Verfahren zur Herstellung dieser Verbindungsklasse weisen häufig Nachteile auf. Beispielsweise erlauben viele der bekannten Verfahren nur die Herstellung mit schlechten Ausbeuten, insbesondere dann, wenn schwerlösliche heterocyclische Aminkomponenten benötigt werden. Andere Verfahren sind unerwünscht vielstufig oder benötigen teilweise hochgiftige Chemikalien (wie z.B. Phosgen) bzw. gefährliche Reagenzien (wie z.B. Alkalihydride), so daß aus sicherheitstechnischen Gründen für die Durchführung im großtechnischen Maßstab besondere Vorkehrungen getroffen werden müssen.

Nach einer dieser im Prinzip bekannten Methoden werden die Sulfonylharnstoffherbizide durch Umsetzung von Sulfonamiden A-SO₂-NH₂ und Carbamaten Ar-O-CO-NR-B erhalten, wobei Ar einen aromatischen Rest bedeutet und A, B und R die bei Formel (I) genannten Bedeutungen haben (vgl. beispielsweise die genannten Patentpublikationen). Bezieht man die Herstellung der Carbamate ein, ist diese Variante meist deshalb nachteilig, weil schlecht zu handhabende, starke Basen eingesetzt werden müssen. Beispielsweise erfordert das aus EP-A-562576 bekannte "Carbamat"-Verfahren zur Herstellung von Sulfonylharnstoffherbiziden einen hohen sicherheitstechnischen Aufwand, weil darin Natriumhydrid als Base eingesetzt wird.

Es wurde nun ein neues Verfahren zur Herstellung der Carbamate und, unter Verwendung der Carbamate, ein Eintopfverfahren gefunden, demgemäß Verbindungen der Formel (I) durch Umsetzung leicht zugänglicher Ausgangsmaterialien überraschend effektiv hergestellt werden können. Im Vergleich zum Stand der Technik aus EP-A-562576 besteht der Vorteil der neuen, erfindungsgemäßen Verfahren z. B. darin, daß Reagentien wie Alkalimetallhydride vermieden werden, die im großtechnischen Maßstab nur mit aufwendigen Maßnahmen sicher zu handhaben sind.

Gegenstand der Erfindung ist ein Verfahren zur Herstellung eines Carbamats der Formel (IV),

Ar-O-CO-NR-B (IV)

worin
- Ar: unsubstituiertes oder substituiertes Aryl, vorzugsweise Phenyl,
- B: einen Pyrimidin-2-yl- oder 1,3,5-Triazin-2-yl-rest, der unsubstituiert oder substituiert ist, und
- R: Wasserstoff, C₁-C₅-Alkyl, C₁-C₅-Haloalkyl, C₁-C₅-Alkoxy oder C₁-C₅-Haloalkoxy bedeuten,
dadurch gekennzeichnet, daß man eine Verbindung der Formel (II),

H-NR-B (II)

mit einer Base M^{⊕}Base^{⊖}, worin M^{⊕} das Äquivalent eines Kations und Base^{⊖} das Anionäquivalent einer Sauerstoffbase bedeutet,
in das entsprechende Salz der Formel (II-S) überführt,

M^{⊕ ⊖}NR-B (II-S)

und die erhaltene Reaktionsmischung mit einem Diarylcarbonat der Formel (III),

(Ar-O)₂CO (III)

unter Entstehen der Verbindung (IV) umsetzt,
wobei in den Formeln (II), (II-S) und (III) die Symbole Ar, B und R wie in Formel (IV) definiert sind.

Das nach dem Verfahren erhaltene Carbamat (IV) kann nach üblichen Methoden zwischenisoliert oder direkt weiterverarbeitet werden. Die direkte Weiterverarbeitung ist überraschenderweise mit guten Ausbeuten und ohne aufwendige Verfahrensmaßnahmen wie Wechsel des Lösungsmittels oder teilweiser Entfernung von Nebenprodukten oder Hilfstoffen aus den vorhergehenden Verfahrensstufen möglich.

Gegenstand der Erfindung ist deshalb auch ein Eintopfverfahren zur Herstellung eines Sulfonylharnstoffs der genannten Formel (I) oder dessen Salze, dadurch gekennzeichnet, daß man eine Verbindung der Formel (II)

H-NR-B (II)

mit einer Base M^{⊕}Base^{⊖}, worin M^{⊕} das Äquivalent eines Kations und Base^{⊖} das Anionäquivalent einer Sauerstoffbase bedeutet,
in das entsprechende Salz der Formel (II-S) überführt,

M^{⊕ ⊖}NR-B (II-S)

die erhaltene Reaktionsmischung mit einem Diarylcarbonat der Formel (III),

(Ar-O)₂CO (III)

worin
Ar unsubstituiertes oder substituiertes Aryl, vorzugsweise Phenyl, bedeutet, umsetzt, wobei das Carbamat der Formel (IV) entsteht,

Ar-O-CO-NR-B (IV)

und die erhaltene Reaktionsmischung mit einem Sulfonamid der Formel (V) umsetzt

A-SO₂NH₂ (V)

und gegebenenfalls anschließend die Verbindung der Formel (I) oder deren Salz isoliert,
wobei in den Formeln (II), (II-S), (IV) und (V) die Symbole A, B und R wie in Formel (I) definiert sind.
Das erfindungsgemäße Verfahren bzw. das Eintopfverfahren wird in der Regel so durchgeführt, daß man zunächst die heterocyclischen Amine der Formel (II) mit geeigneten Sauerstoffbasen M^{⊕}Base^{⊖} (Oxiden, Hydroxiden, Carbonaten oder Alkoholaten) in einem Lösungsmittel unter Bildung des Salzes (II-S) deprotoniert.
Geeignete Salze von Sauerstoffbasen M^{⊕}Base^{⊖} sind beispielsweise Salze aus der Gruppe Alkalimetalloxide, Erdalkalimetalloxide, Alkalimetallhydroxide, z. B. NaOH und KOH, Erdalkalimetallhydroxide, Tetraalkylammoniumhydroxide, Alkalimetallalkoholate, z. B. Natriumethylat, Natriummethylat, Kaliumethylat, Kaliummethylat, Natriumisopropylat, Kaliumisopropylat, Natrium-tert.-butylat, Kalium-tert.-butylat und ähnliche verzweigte Alkoholate, und Erdalkalimetallalkoholate, vorzugsweise Alkalimetallalkoholate des Natriums und Kaliums.

Als Lösungsmittel eignen sich solche, die in der Lage sind, die Reaktanten bei den gewählten Temperaturen zu lösen bzw. zumindest anzulösen. Die geeigneten Lösungsmittel lassen sich in Vorversuchen leicht herausfinden. Von besonderem Interesse sind unter den Reaktionsbedingungen inerte organische Lösungsmittel, vor allem aprotisch dipolare Lösungsmittel sowie deren Mischungen.
Beispiele für prinzipiell geeignete Lösungsmittel sind
- Ether, wie Diethylether, Tetrahydrofuran (THF), Dioxan, Diglyme und Tetraglyme,
- Amide, wie Dimethylformamid (DMF), Dimethylacetamid (DMA) und N-Methylpyrrolidon,
- Ketone, wie Aceton,
- Nitrile, wie Acetonitril, Propionitril, Butyronitril und Benzonitril,
- Sulfoxide und Sulfone, wie Dimethylsulfoxid (DMSO) und Sulfolan,
- halogenierte aliphatische und aromatische Kohlenwasserstoffe, wie Methylenchlorid und Chlorbenzol.

Die Bildung des Salzes (II-S) kann in einem weiten Temperaturbereich erfolgen, der im Einzelfall vom Schmelzpunkt oder Siedepunkt des verwendeten Lösungsmittels sowie dessen Löseeigenschaften begrenzt sein kann. In der Regel liegt die Temperatur bei der Salzbildung im Bereich von -40 °C bis zum Siedepunkt des jeweiligen Lösungsmittels, vorzugsweise im Bereich von -20 °C bis zum Siedepunkt des jeweiligen Lösungsmittels, insbesondere von -10 °C bis + 100 °C.

Das reaktionsfähige Anion im Salz (II-S) des heterocyclischen Amins wird anschließend vorzugsweise ohne Zwischenisolierung mit einem Diarylcarbonat (III), wie z.B. Diphenylcarbonat, zum entsprechenden Carbamat (IV) umgesetzt. Diese Reaktion kann in einem weiten Temperaturbereich in Abhängigkeit vom Lösungsmittel durchgeführt werden und erfolgt beispielsweise bei Temperaturen von -40 bis + 180°C, vorzugsweise von -20 bis + 150°C, mehr bevorzugt von -10 bis 100°C, insbesondere von 0 bis 100°C.

Zur Durchführung der Reaktion des Anions (II-S) und des Diarylcarbonats (III) kann von Fall zu Fall die Zugabe von Trockenmittel, z.B. Molekularsieb (3Å bzw. 4Å), oder die Zugabe von Phasentransferkatalysatoren, z.B. Polyethylenglykolether, organische Ammoniumsalze, Kronenether usw., vorteilhaft sein.

Das Carbamat (IV) kann anschließend nach üblichen, beispielsweise kombinierten schonenden Methoden wie Filtration der Lösung, Einrotieren, Umkristallisation, Extraktion bei verschiedenen pH-Werten, Chromatographie etc. isoliert werden. So kann beispielsweise 4-Methoxy-6-methyl-2-phenoxycarbonylamino-1,3,5-triazin in zufriedenstellender Reinheit und Ausbeute gewonnen werden (¹H-NMR (200 Mhz, D₆-DMSO): δ ppm = 8,5 (s,1H), 7,4 (m,2H), 7,2 (m,3H), 4,05 (s,3H)).

Die optimalen Verhältnisse der Komponenten heterocyclisches Amin (II), M^{⊕}Base^{⊖} und Diarylcarbonat (III), können von Fall zu Fall variieren. Beispielsweise wird die Base im Verhältnis zum Amin (II) zweckmäßig äquimolar oder im Überschuß eingesetzt, beispielsweise im molaren Verhältnis 1:1 bis 5:1, vorzugsweise 1:1 bis 3:1. Dabei wird das Diarylcarbonat (III) im Verhältnis zum Amin (II) beispielsweise äquimolar, im Überschuß oder im Unterschuß eingesetzt, vorzugsweise im molaren Verhältnis 1:2 bis 2:1, insbesondere 1:1,5 bis 1,5:1.

Das intermediär gebildete Carbamat (IV) wird nach der erfindungsgemäßen Eintopfvariante ohne Zwischenisolierung mit dem Sulfonamid der Formel (V) umgesetzt. Die Reaktionstemperaturen sind dabei in der Regel von -40 bis 180°C, vorzugsweise von -20 bis 150°C, insbesondere von 0 bis 80°C.

Nach beendeter Reaktion können wahlweise die Sulfonylharnstoffe der Formel (I) oder deren Salze (I-S) nach den üblichen Reinigungsschritten isoliert werden.

Zur Gewinnung der neutralen Sulfonylharnstoffe wird beispielsweise zunächst das Reaktionsgemisch filtriert und dann das Filtrat vorsichtig in eine verdünnte wäßrige Säure, wie z.B. verdünnte Salzsäure oder Ameisensäure, gegossen. Nach dem Waschen des abgeschiedenen Produkts - beispielsweise mit Wasser und/oder einem oder mehreren organischen Solventien (z.B. Alkohole wie Methanol, Ethanol und Isopropanol; Ether wie Diethylether und tert.-Butylmethylether; Ketone wie Aceton; Ester wie Ethylacetat und Methylacetat) und dem Trocknen können die gewünschten Produkte in guten Ausbeuten und guten Reinheiten (in der Regel mehr als 90 %ig) erhalten werden.
Alternativ kann das Filtrat in ein Zweiphasensystem aus einem organischen Solvens, wie z.B. Toluol, tert.-Butylmethylether, Methyl- oder Ethylacetat, und verdünnter wäßriger Säure eingetragen werden. Der abgeschiedene Sulfonylharnstoff fällt dann direkt mit guten Reinheiten und Ausbeuten an.

Zur Gewinnung der Salze der Verbindungen (I) erfolgt die Isolierung des Produktes, ohne zuvor das Reaktionsgemisch anzusäuern. Die Salze der Sulfonylharnstoffe der Formel (I) können beispielsweise auf folgendem Weg isoliert werden:

Nach beendeter Reaktion und Filtration des Gemisches wird zum Filtrat ein unpolares organisches Solvens zugesetzt, um die Löslichkeit des Salzes in dem Reaktionsgemisch zu erniedrigen. Alternativ kann das Reaktionsgemisch durch Abdestillieren flüchtiger Komponenten aufkonzentriert werden. Das danach abgeschiedene Sulfonylharnstoffsalz kann beispielsweise durch Verrühren mit einem organischen Solvens, wie z.B. Methanol, Diethylether, Ethylacetat, Methylacetat, Ethanol, tert.-Butylmethylether oder einem Solvensgemisch, gereinigt werden.

Zur Durchführung der Reaktion des Carbamats (IV) und des Sulfonamids (V) kann von Fall zu Fall die Zugabe von Trockenmittel, z.B. Molekularsieb (3Å bzw. 4Å) oder die Zugabe von Phasentransferkatalysatoren, z.B. Polyethylenglykolether, organischen Ammoniumsalzen, Kronenether usw., vorteilhaft sein.

Das Verhältnis der Komponenten Sulfonamid (V), Diarylcarbonat (III), heterocyclisches Amin (II) und Base M^{⊕}Base^{⊖} kann von Fall zu Fall variieren. Vorzugsweise ist das molare Mengenverhältnis von Sulfonamid (V) und Komponenten der Formel (III) bzw. (II) jeweils von 1:0,8 bis 1:3,5, insbesondere jeweils von 1:1 bis 1:2.

In Formel (I) und allen nachfolgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt.
Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl.

Ein aromatischer carbocyclischer Rest (= Aryl) bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.
Ein heterocyclischer Rest oder Ring (Heterocyclyl) kann gesättigt, ungesättigt oder heteroaromatisch sein; er enthält vorzugsweise ein oder mehrere Heteroatome im Ring, welche auch substituierte Heteroatome einschließen, vorzugsweise aus der Gruppe N, O, S, SO, SO₂; beispielsweise ist er ein aliphatischer Heterocyclylrest mit 3 bis 7 Ringatomen oder ein heteroaromatischer Rest mit 5 oder 6 Ringatomen und enthält 1, 2 oder 3 Heteroeinheiten. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl, oder ist ein partiell oder vollständig hydrierter Rest wie Oxiranyl, Pyrrolidyl, Piperidyl, Piperazinyl, Dioxolanyl, Morpholinyl, Tetrahydrofuryl.

Als Substituenten für einen substituierten aromatischen oder heteroaromatischen Rest kommen beispielsweise in Frage:
Ein oder mehrere, vorzugsweise 1, 2 oder 3 Reste aus der Gruppe Halogen, Alkyl, Haloalkyl, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Haloalkylsulfinyl, Alkylsulfonyl und Haloalkylsulfonyl; im Begriff "substituierte Reste" wie substituiertes Aryl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, C₁-C₄-Alkyl, vorzugsweise Methyl oder Ethyl, C₁-C₄-Haloalkyl, vorzugsweise Trifluormethyl, C₁-C₄-Alkoxy, vorzugsweise Methoxy oder Ethoxy, C₁-C₄-Haloalkoxy, Nitro und Cyano. Besonders bevorzugt sind dabei die Substituenten Methyl, Methoxy und Chlor.

Für das erfindungsgemäße Verfahren eignen sich als Verbindungen der Formel (II) primäre oder sekundäre Aminopyrimidine bzw. -triazine, wobei die heterocyclischen Ringe unsubstituiert oder substituiert sein können. Typische Amine stellen z.B. Verbindungen der Formel (lla) dar, 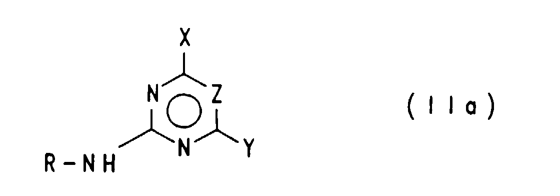 worin
- R: Wasserstoff, C₁-C₅-Alkyl, C₁-C₅-Haloalkyl, C₁-C₅-Alkoxy oder C₁-C₅-Haloalkoxy, vorzugsweise Wasserstoff und C₁-C₄-Alkyl,
- X, Y: unabhängig voneinander Wasserstoff, Halogen, C₁-C₆-Alkyl, C₁-C₆-Alkoxy, C₁-C₆-Alkylthio, Mono- und Di-(C₁-C₄-alkyl)amino, wobei jeder der sechs letztgenannten Reste im Alkylteil unsubstituiert oder durch einen oder mehrere Reste aus der Gruppe Halogen, C₁-C₄-Alkoxy, C₁-C₄-Haloalkoxy und C₁-C₄-Alkylthio substituiert ist, oder C₃-C₆-Cycloalkyl, C₂-C₆-Alkenyl, C₂-C₆-Alkinyl, C₂-C₆-Alkenyloxy oder C₂-C₆-Alkinyloxy und
- Z: CH oder N bedeuten.

Substituierte oder unsubstituierte Diarylcarbonate der Formel (III), insbesondere Diphenylcarbonate, sind als Reagenz für das erfindungsgemäße Verfahren geeignet. Bevorzugte Verbindungen entsprechen der Formel (Illa), 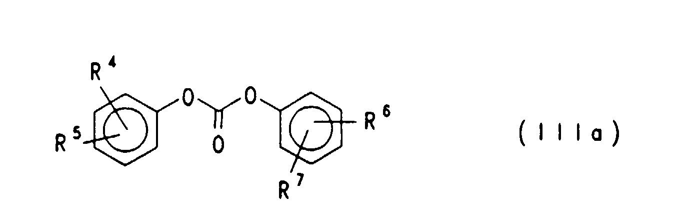 worin
R⁴, R⁵, R⁶ und R⁷ unabhängig voneinander Wasserstoff, Halogen, C₁-C₄-Alkyl, C₁-C₄-Haloalkoxy, C₁-C₄-Haloalkyl, C₁-C₄-Alkoxy, Nitro oder CN bedeuten.

Als aromatische oder heteroaromatische Sulfonamide der Formel A-SO₂NH₂ (V) sind Verbindungen der Formeln (Va) bis (Ve) bevorzugt, 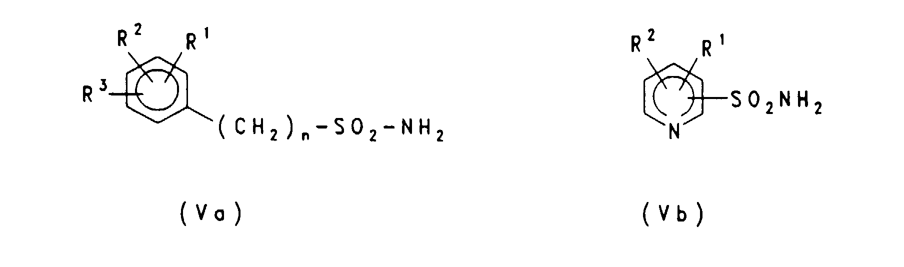 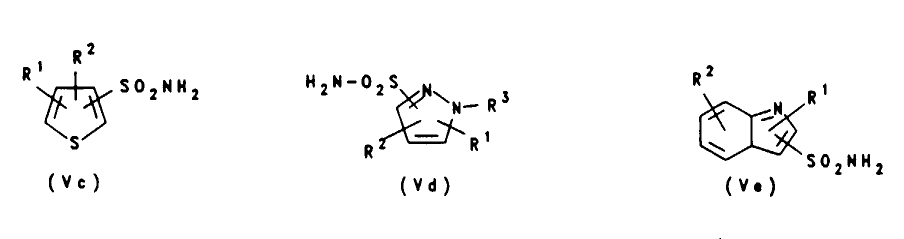 worin
- n: 0 oder 1,
- R¹: H, OH, CO₂H, CONH₂, Halogen, CN, NO₂, NH₂, SO₂NH₂, C₁-C₆-Alkyl, C₂-C₆-Alkenyl, C₂-C₆-Alkinyl, C₃-C₇-Cycloalkyl, C₄-C₁₀-Cycloalkylalkyl, C₁-C₆-Alkoxy, C₂-C₆-Alkenoxy, C₂-C₆-Alkinoxy, C₃-C₇-Cycloalkoxy, C₄-C₁₀-Cycloalkylalkoxy, Mono- oder Di-(C₁-C₄-alkyl)amino, C₁-C₆-Alkylthio, C₁-C₆-Alkylsulfinyl, C₁-C₆-Alkylsulfonyl, C₂-C₆-Alkenylsulfinyl, C₂-C₆-Alkenylsulfonyl, C₂-C₆-Alkanylthio, C₂-C₆-Alkinylthio, C₂-C₆-Alkinylsulfinyl, C₂-C₆-Alkinylsulfonyl, C₃-C₇-Cycloalkylsulfonyl, C₄-C₉-Cycloalkylalkylsulfonyl, C₁-C₆-Alkoxycarbonyl, C₂-C₆-Alkenoxycarbonyl, C₂-C₆-Alkinoxycarbonyl, C₃-C₇-Cycloalkoxycarbonyl, Mono- und Di-(C₁-C₄-alkyl(aminocarbonyl, Mono- und Di-(C₁-C₄-alkyl)aminosulfonyl, wobei jeder der 31 letztgenannten Reste unsubstituiert oder durch einen oder mehrere Reste aus der Gruppe Halogen, CN, OH, NH₂, NO₂, NHCH₃, N(CH₃)₂, C₁-C₃-Alkoxy, C₁-C₃-Haloalkoxy, C₁-C₃-Alkylthio, C₁-C₃-Haloalkylthio, Phenyl, COOCH₃, COOC₂H₅, CONH₂, CONHCH₃, CON(CH₃)₂, SO₂CH₃, SO₂C₂H₅, SOCH₃ und SOC₂H₅ substituiert ist,
- R²: H, OH, NH₂, Mono- und Di-(C₁-C₄-alkyl)amino, C₁-C₄-Alkoxy, C₂-C₄-Alkenoxy, C₂-C₄-Alkinoxy, wobei die letztgenannten 5 Reste unsubstituiert oder durch einen oder mehrere Reste aus der Gruppe Halogen, CN, C₁-C₃-Haloalkyl, C₁-C₃-Alkoxy und C₁-C₃-Haloalkoxy substituiert sind, oder NR'R", CH₂NR'R", Halogen, CN, NO₂ oder Heterocyclyl,
wobei
R' H, OH, C₁-C₆-Alkyl, C₁-C₅-Alkoxy, C₃-C₇-Cycloalkyl, C₄-C₈-Cycloalkylalkyl, wobei die 4 letztgenannten Reste unsubstituiert oder durch einen oder mehrere Reste aus der Gruppe Halogen, C₁-C₃-Haloalkyl, C₁-C₃-Alkoxy und C₁-C₃-Haloalkoxy substituiert sind, und
R" einen Acylrest, wie z.B. CO-NH₂, CHO, C₁-C₆-Alkylcarbonyl, C₁-C₆-Alkoxycarbonyl, C₃-C₇-Cycloalkylcarbonyl, C₄-C₉-Cycloalkylcarbonyl, C₃-C₇-Cycloalkoxycarbonyl, C₄-C₉-Cycloalkylalkoxycarbonyl, C₁-C₆-Alkylsulfonyl, Mono- und Di-(C₁-C₄-alkyl)aminocarbonyl, Mono- und Di-(C₁-C₄-alkyl)aminosulfonyl, wobei jeder der 11 letztgenannten Reste unsubstituiert oder durch einen oder mehrere Reste aus der Gruppe Halogen, C₁-C₃-Alkoxy und C₁-C₃-Haloalkoxy substituiert ist, oder NR'R" einen substituierten oder unsubstituierten Hydrazinrest und
- R³: H, Halogen, C₁-C₃-Alkyl, C₁-C₃-Haloalkyl, C₁-C₃-Alkoxy, C₁-C₃-Haloalkoxy, NH₂, NHCH₃, N(CH₃)₂, NO₂ oder CN
bedeuten.

Das erfindungsgemäße Verfahren ist in Schema 1 am Beispiel bestimmter Pyrimidine und Triazine der Formel (II) zusammengefaßt.

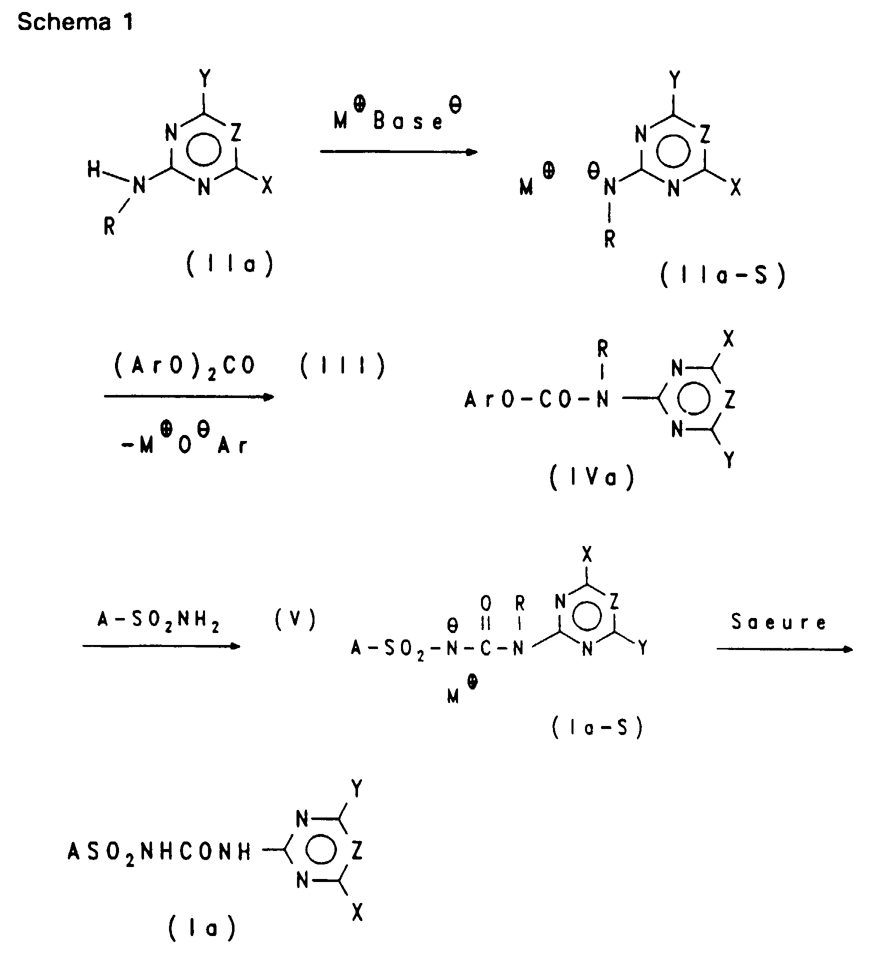

Zur Synthese von Sulfonylharnstoffen der Formel (I) nach dem erfindungsgemäßen Verfahren werden die obengenannten Nachteile, wie z.B. Verwendung hochgiftiger bzw. gefährlicher Chemikalien (z.B. Phosgen bzw. Alkalihydride wie Natriumhydrid) umgangen. Weiterhin können damit auch schwerlösliche heterocyclische Amine - wie z.B. 2-Amino-4-methoxy-6-methyltriazin - in sehr guten Ausbeuten zu Sulfonylharnstoffen umgesetzt werden.

In den nachfolgenden Beispielen beziehen sich Mengenangaben und Mengenverhältnisse (wie Prozentangaben) auf das Gewicht, wenn nicht andere Definitionen angegeben sind.

### Beispiel 1

### 2-[[[[(4-Methoxy-6-methyl-1,3,5-triazin-2-yl)-amino]-carbonyl]-amino]-sulfonyl]-4-iodbenzoesäuremethylester

2,85 g 2-Amino-4-methoxy-6-methyl-1,3,5-triazin wird in 40 ml Dimethylacetamid (DMA) vorgelegt und mit 2,22 g Natrium-tert.-butylat versetzt. Nach 30 min Rühren kühlt man auf 5°C ab und setzt 4,94 g Diphenylcarbonat hinzu. Nach 1 h Rühren bei Raumtemperatur wird dieses Gemisch zu einer Lösung aus 5,00 g 2-Amino-sulfonyl-4-iodbenzoesäuremethylester (92,5 %ig) und 15 ml DMA bei 0°C zugetropft. Nach 2 h Rühren bei Raumtemperatur werden Feststoffe durch Filtration aus dem Reaktionsgemisch abgetrennt. Das Filtrat wird in ein Gemisch aus 200 ml Eiswasser und 10 ml konzentrierte Salzsäure eingetragen. Der abgeschiedene Harnstoff wird mit Wasser, Methanol und Diisopropylether gewaschen und anschließend getrocknet.
Man erhält so den gewünschten Sulfonylharnstoff in einer Ausbeute von 70% (5,2 g; Reinheit: 93,3 % (HPLC));
¹H-NMR (D₆-DMSO, 200 MHz) δ (ppm) = 12,6 (s, 1H), 11,2 (s, 1H), 8,4 (d, 1H), 8,2 (dd, 1H), 7,6 (d, 1H), 4,0 (s, 3H), 3,8 (s, 3H), 2,5 (s, 3H).

### Beispiel 2

### 2-[[[[(4-Methoxy-6-methyl-1,3,5-triazin-2-yl)-amino]-carbonyl]-amino]-sulfonyl]-4-iodbenzoesäuremethylester

Zu einer Suspension aus 2,85 g 2-Amino-4-methoxy-6-methyl-1,3,5-triazin in 40 ml Dimethylacetamid (DMA) werden bei Raumtemperatur 2,59 g Kaliumtert.-butylat zugesetzt. Anschließend wird bei ca. 5 °C zu dieser Mischung eine Lösung aus 4,94 g Diphenylcarbonat in 20 ml DMA zugetropft. Das erhaltene Gemisch wird anschließend bei ca. 5 °C zu einer Lösung aus 5,00 g 2-Aminosulfonyl-4-iodbenzoesäure-methylester (92,5 %ig) in 15 ml DMA zugetropft. Nach beendeter Reaktion wird das Gemisch über Kieselgur (®Celite) filtriert. Das Filtrat wird in eine Lösung aus 200 ml Eiswasser und 10 ml konzentrierter Salzsäure eingetragen. Das abgeschiedene Rohprodukt wird anschließend durch Verrühren mit Methanol und Diisopropylether gereinigt und getrocknet. Ausbeute 4,50 g (66% d.Th.).

### Beispiel 3

### 2-[[[[(4-Methoxy-6-methyl-1,3,5-triazin-2-yl)-amino]-carbonyl]-amino]-sulfonyl]-4-iodbenzoesäuremethylester

Zu einer Suspension aus 1,05 g 2-Amino-4-methoxy-6-methyl-1,3,5-triazin in 20 ml Dimethylacetamid (DMA) werden bei Raumtemperatur 0,96 g Natriumtert.-butylat unter kräftigem Rühren zugesetzt. Innerhalb von 7 min wird anschließend unter Eisbadkühlung eine Lösung aus 1,12 g Diphenylcarbonat in 10 ml DMA zugesetzt. Man rührt 15 min bei dieser Temperatur nach und tropft anschließend innerhalb von 7 min eine Lösung aus 1,84 g 2-Aminosulfonyl-4-iodbenzoesäure-methylester (92,5 %ig) in DMA zu. Nach beendeter Reaktion wird der Ansatz wie unter Beispiel 1 beschrieben aufgearbeitet. Man erhält so 1,47 g an gewünschtem Produkt (58% d. Th.).

### Beispiel 4

### 2-[[[[(4-Methoxy-6-methyl-1,3,5-triazin-2-yl)amino]carbonyl]amino]sulfonyl]-4-iodbenzoesäuremethylester

Zu einer Suspension aus 3,69 g 2-Amino-4-methoxy-6-methyl-1,3,5-triazin in 100 ml DMA werden bei Raumtemperatur 5,09 g Natrium-tert.-butylat zugesetzt. Nach dem Abkühlen auf 3-7°C wird eine Lösung aus 5,64 g Diphenylcarbonat und 50 ml DMA zugetropft. Das Reaktionsgemisch wird 15 min bei dieser Temperatur nachgerührt. Anschließend wird das erhaltene Reatkionsgemisch zu einer Lösung aus 8,85 g 2-Aminosulfonyl-4-iodbenzoesäuremethylester und 50 ml DMA bei 3-7°C zugetropft. Nachdem das Reaktionsgemisch 1 h bei 3°C und 2 h bei Raumtemperatur nachgerührt worden ist, werden die flüchtigen Komponenten unter reduziertem Druck abdestilliert. Der Rückstand wird in 250 ml Wasser gelöst und mit konzentrierter Salzsäure angesäuert (pH = 2-3). Das abgeschiedeneRohprodukt wird mit Methanol und Diisopropylether gewaschen. Nach dem Trocknen werden 8,4 g des gewünschten Produktes (Reinheit > 92 %) gewonnen.

### Beispiel 5

### 2-[[[[(4-Chlor-6-methoxypyrimidin-2-yl)amino]carbonyl]amino]sulfonyl]benzoesäuremethylester

Zu einer Suspension aus 1,68 g 2-Amino-4-chlor-6-methoxypyrimidin in 30 ml DMA werden 2,03 g Natrium-tert.-butylat zugesetzt. Nach 30 min Rühren bei Raumtemperatur wird das Reaktionsgemisch bei 3-7°C einer Lösung aus 1,68 g Diphenylcarbonat und 25 ml DMA zugetropft. Das Reaktionsgemisch läßt man langsam auf Raumtemperatur kommen und dosiert anschließend dieses Gemisch bei 3-7°C zu einer Lösung aus 2,03 g 2-Aminosulfonylbenzoesäuremethylester und 20 ml DMA. Nach 2 h Rühren bei dieser Temperatur wird das Reaktionsgemisch unter reduziertem Druck aufkonzentriert, der Rückstand wird in Wasser aufgenommen mit tert.-Butylmethylether versetzt und mit konzentrierter Salzsäure angesäuert. Der ausgefallene Feststoff wird abgetrennt und mit tert.-Butylmethylether gewaschen und getrocknet.
Man erhält so 2,36 g der gewünschten Verbindung (Reinheit > 90 %); ¹H-NMR (D₆-DMSO, 200 MHz) δ (ppm) = 12,0 (s, 1H), 10,9 (s, 1H), 8,2 (m, 1H), 7,8 (m, 3H), 6,9 (s, 1H), 4,0 (s, 3H), 3,8 (s, 3H).

### Vergleichsbeispiel

### 2-[[[[(4-Methoxy-6-methyl-1,3,5-triazin-2-yl)-amino]-carbonyl]-amino]-sulfonyl]-4-iodbenzoesäuremethylester

Zu einer Suspension aus 0,23 g 2-Amino-4-methoxy-6-methyl-1,3,5-triazin in 3 ml Dimethylacetamid (DMA) werden bei Raumtemperatur 0,084 g Natriumhydrid (60%ig in Paraffinöl) zugesetzt und gerührt, bis die Gasentwicklung beendet ist. Zu dieser Lösung wird anschließend bei ca. 5 °C langsam eine Lösung aus 0,44 g Diphenylcarbonat in 1 ml DMA zugetropft. Nach 10 min Rühren wird unter Eisbadkühlung eine Lösung aus 0,68 g 2-Aminosulfonyl-4-iodbenzoesäure-methylester (92,5 %ig) in 2 ml DMA langsam zugetropft. Nach beendeter Reaktion wird das Reaktionsgemisch anschließend in eine Lösung aus 30 ml Eiswasser und 0,25 ml konzentrierter Salzsäure eingetragen. Das abgeschiedene Rohprodukt wird anschließend durch Waschen mit wenig Wasser, Methanol und Diisopropylether gereinigt und getrocknet. Ausbeute 0,47 g (46% d. Th.).

## Patentansprüche

1. Verfahren zur Herstellung einer Verbindung der Formel (IV),
Ar-O-CO-NR-B (IV)
worin
Ar unsubstituiertes oder substituiertes Aryl,
B einen Pyrimidin-2-yl- oder 1,3,5-Triazin-2-yl-rest, der unsubstituiert oder substituiert ist, und
R Wasserstoff, C₁-C₅-Alkyl, C₁-C₅-Haloalkyl, C₁-C₅-Alkoxy oder C₁-C₅-Haloalkoxy
bedeuten,
dadurch gekennzeichnet, daß man eine Verbindung der Formel (II),
H-NR-B (II)
mit einer Base M^{⊕}Base^{⊖}, worin M^{⊕} das Äquivalent eines Kations und Base^{⊖} das Anionäquivalent einer Sauerstoffbase bedeutet,
in das entsprechende Salz der Formel (II-S) überführt,
M^{⊕ ⊖}NR-B (II-S)
und die erhaltene Reaktionsmischung mit einem Diarylcarbonat der Formel (III),
(Ar-O)₂CO (III)
unter Entstehen der Verbindung (IV) umsetzt,
wobei in den Formeln (II), (II-S) und (III) die Symbole Ar, B und R wie in Formel (IV) definiert sind.

2. Verfahren nach Anspruch 1, dadurch gekennzeichnet, daß man als Verbindung der Formel (II) eine Verbindung der Formel (IIa) einsetzt, 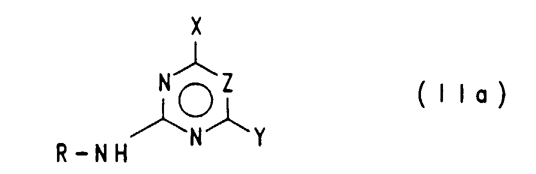 worin
R Wasserstoff, C₁-C₅-Alkyl, C₁-C₅-Haloalkyl, C₁-C₅-Alkoxy oder C₁-C₅-Haloalkoxy,
X, Y unabhängig voneinander Wasserstoff, Halogen, C₁-C₆-Alkyl, C₁-C₆-Alkoxy, C₁-C₆-Alkylthio, Mono- und Di-(C₁-C₄-alkyl)amino, wobei jeder der sechs letztgenannten Reste im Alkylteil unsubstituiert oder durch einen oder mehrere Reste aus der Gruppe Halogen, C₁-C₄-Alkoxy, C₁-C₄-Haloalkoxy und C₁-C₄-Alkylthio substituiert ist, oder C₃-C₆-Cycloalkyl, C₂-C₆-Alkenyl, C₂-C₆-Alkinyl, C₂-C₆-Alkenyloxy oder C₂-C₆-Alkinyloxy und
Z CH oder N bedeuten.

3. Verfahren nach Anspruch 1 oder 2, dadurch gekennzeichnet, daß man als Verbindung der Formel (III) eine Verbindung der Formel (llla) einsetzt, 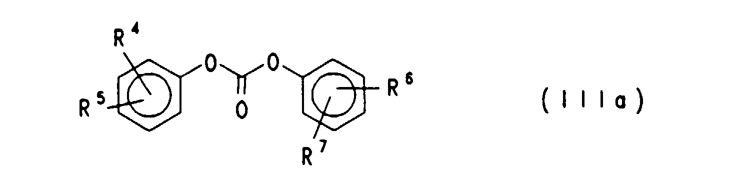 worin
R⁴, R⁵, R⁶ und R⁷ unabhängig voneinander Wasserstoff, Halogen, C₁-C₄-Alkyl, C₁-C₄-Haloalkoxy, C₁-C₄-Haloalkyl, C₁-C₄-Alkoxy oder CN bedeuten.

4. Verfahren nach einem der Ansprüche 1 bis 3, dadurch gekennzeichnet, daß man als Base M^{⊕}Base^{⊖} Salze aus der Gruppe der Alkalimetallalkoholate einsetzt.

5. Verfahren nach einem der Ansprüche 1 bis 4, dadurch gekennzeichnet, daß man als Base M^{⊕}Base^{⊖} Salze aus der Gruppe der Alkalimetallalkoholate in einem aprotischen dipolaren Lösungsmittel einsetzt.

6. Verfahren nach Anspruch 5, dadurch gekennzeichnet, daß man die Base bei Temperaturen von -40°C bis zum jeweiligen Siedepunkt des Lösungsmittels einsetzt.

7. Verfahren nach einem der Ansprüche 1 bis 6, dadurch gekennzeichnet, daß man die Umsetzung zum Carbamat (IV) bei Temperaturen von -40 bis 180°C durchführt.

8. Verfahren nach einem der Ansprüche 1 bis 7, dadurch gekennzeichnet, daß man die erhaltene Reaktionsmischung, welche die Verbindung der Formel (IV) enthält, mit einem Sulfamid der Formel (V) zur Verbindung der Formel (I) oder deren Salz umsetzt,
Ar-O-CO-NR-B (IV)
A-SO₂NH₂ (V)
A-SO₂-NH-CO-NR-B (I)
wobei in den Formeln (IV), (V) und (I) A einen organischen Rest, der gegebenenfalls Heteroatome enthält, bedeutet und die Symbole Ar, A, B und R in Formel (I) wie in Formel (IV) definiert sind,
und gegebenenfalls anschließend die Verbindung der Formel (I) oder deren Salz isoliert.

9. Verfahren zur Herstellung einer Verbindung der Formel (I) oder deren Salze,
A-SO₂-NH-CO-NR-B (I)
worin
A einen organischen Rest, der gegebenenfalls Heteroatome enthält,
B einen Pyrimidin-2-yl- oder 1,3,5-Triazin-2-yl-rest, der unsubstituiert oder substituiert ist, und
R Wasserstoff, C₁-C₅-Alkyl, C₁-C₅-Haloalkyl, C₁-C₅-Alkoxy oder C₁-C₅-Haloalkoxy
bedeuten,
dadurch gekennzeichnet, daß man eine Verbindung der Formel (II)
H-NR-B (II)
mit einer Base M^{⊕}Base^{⊖}, worin M^{⊕} das Äquivalent eines Kations und Base^{⊖} das Anionäquivalent einer Sauerstoffbase bedeutet,
in das entsprechende Salz der Formel (II-S) überführt,
M^{⊕ ⊖}NR-B (II-S)
die erhaltene Reaktionsmischung mit einem Diarylcarbonat der Formel (III),
(Ar-O)₂CO (III)
worin
Ar unsubstituiertes oder substituiertes Aryl bedeutet,
umsetzt, wobei das Carbamat der Formel (IV) entsteht,
Ar-O-CO-NR-B (IV)
und die erhaltene Reaktionsmischung mit einem Sulfonamid der Formel (V) umsetzt
A-SO₂NH₂ (V)
und gegebenenfalls anschließend die Verbindung der Formel (I) oder deren Salz isoliert,
wobei in den Formeln (II), (II-S), (IV) und (V) die Symbole A, B und R wie in Formel (I) definiert sind.

10. Verfahren nach Anspruch 9, dadurch gekennzeichnet, daß man die Umsetzung des Carbamats (IV) mit dem Sulfonamid der Formel (V) bei Temperaturen von -40 bis 180°C durchführt.

## Claims

1. A process for the preparation of a compound of the formula (IV)
Ar-O-CO-NR-B (IV)
in which
Ar is unsubstituted or substituted aryl,
B is a pyrimidin-2-yl or 1,3,5-triazin-2-yl radical, which is unsubstituted or substituted, and
R is hydrogen, C₁-C₅-alkyl, C₁-C₅-haloalkyl, C₁-C₅-alkoxy or C₁-C₅-haloalkoxy,
which comprises converting a compound of the formula (II)
H-NR-B (II)
with a base M^{⊕} Base^{⊖}, in which M^{⊕} is the equivalent of a cation and Base^{⊖} is the anion equivalent of an oxygen base,
into the corresponding salt of the formula (II-S)
M^{⊕ ⊖}NR-B (II-S)
and reacting the resulting reaction mixture with a diaryl carbonate of the formula (III)
(Ar-O)₂CO (III)
to form the compound (IV),
in which, in the formulae (II), (II-S) and (III), the symbols Ar, B and R are defined as in formula (IV).

2. The process as claimed in claim 1, wherein a compound of the formula (IIa) 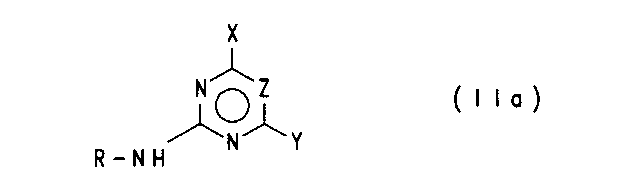 in which
R is hydrogen, C₁-C₅-alkyl, C₁-C₅-haloalkyl, C₁-C₅-alkoxy or C₁-C₅-haloalkoxy,
X and Y independently of one another are hydrogen, halogen, C₁-C₆-alkyl, C₁-C₆-alkoxy, C₁-C₆-alkylthio, mono- or di-(C₁-C₄-alkyl)amino, where each of the last six radicals mentioned is unsubstituted in the alkyl part or substituted by one or more radicals from the group consisting of halogen, C₁-C₄-alkoxy, C₁-C₄-haloalkoxy and C₁-C₄-alkylthio, or C₃-C₆-cycloalkyl, C₂-C₆-alkenyl, C₂-C₆-alkynyl, C₂-C₆-alkenyloxy or C₂-C₆-alkynyloxy and
Z is CH or N,
is employed as the compound of the formula (II).

3. The process as claimed in claim 1 or 2, wherein a compound of the formula (Illa) 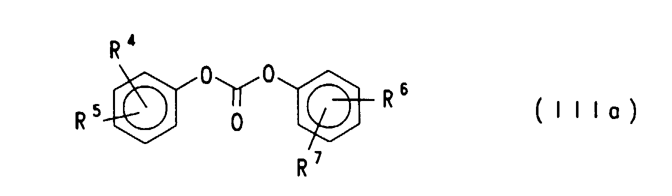 in which
R⁴, R⁵, R⁶ and R⁷ independently of one another are hydrogen, halogen, C₁-C₄-alkyl, C₁-C₄-haloalkoxy, C₁-C₄-haloalkyl, C₁-C₄-alkoxy or CN,
is employed as the compound of the formula (III).

4. The process as claimed in one of claims 1 to 3, wherein a salt from the group consisting of alkali metal alcoholates is employed as the base M^{⊕}Base^{⊖}.

5. The process as claimed in one of claims 1 to 4, wherein a salt from the group consisting of alkali metal alcoholates is employed as the base M^{⊕}Base^{⊖} in an aprotic dipolar solvent.

6. The process as claimed in claim 5, wherein the base is employed at temperatures from -40°C up to the particular boiling point of the solvent.

7. The process as claimed in one of claims 1 to 6, wherein the reaction to give the carbamate (IV) is carried out at temperatures from -40 to 180°C.

8. The process as claimed in one of claims 1 to 7, wherein the resulting reaction mixture, which comprises the compound of the formula (IV), is reacted with a sulfamide of the formula (V) to give the compound of the formula (I) or a salt thereof
Ar-O-CO-NR-B (IV)
A-SO₂NH₂ (V)
A-SO₂-NH-CO-NR-B (I)
in which, in the formulae (IV), (V) and (I), A is an organic radical, which optionally contains heteroatoms, and the symbols Ar, A, B and R in formula (I) are defined as in formula (IV),
and, if appropriate, the compound of the formula (I) or the salt thereof is then isolated.

9. A process for the preparation of a compound of the formula (I) or a salt thereof
A-SO₂-NH-CO-NR-B (I)
in which
A is an organic radical, which optionally contains heteroatoms,
B is a pyrimidin-2-yl or 1,3,5-triazin-2-yl radical, which is unsubstituted or substituted, and
R is hydrogen, C₁-C₅-alkyl, C₁-C₅-haloalkyl, C₁-C₅-alkoxy or C₁-C₅-haloalkoxy,
which comprises converting a compound of the formula (II)
H-NR-B (II)
with a base M^{⊕} Base^{⊖}, in which M^{⊕} is the equivalent of a cation and Base^{⊖} is the anion equivalent of an oxygen base,
into the corresponding salt of the formula (II-S)
M^{⊕⊖} NR-B (II-S),
reacting the resulting reaction mixture with a diaryl carbonate of the formula (III)
(Ar-O)₂CO (III)
in which
Ar is unsubstituted or substituted aryl,
the carbamate of the formula (IV) being formed
Ar-O-CO-NR-B (IV)
and reacting the resulting reaction mixture with a sulfonamide of the formula (V)
A-SO₂NH₂ (V)
and, if appropriate, then isolating the compound of the formula (I) or salt thereof, in which, in the formulae (II), (II-S), (IV) and (V), the symbols Ar, B and R are defined as in formula (I).

10. The process as claimed in claim 9, wherein the reaction of the carbamate (IV) with the sulfonamide of the formula (V) is carried out at temperatures from -40 to 180°C.

## Revendications

1. Procédé pour la préparation d'un composé de formule (IV),
Ar-O-CO-NR-B (IV)
dans laquelle
Ar représente un groupe aryle non substitué ou substitué
B représente un groupe pyrimidin-2-yle ou 1,3,5-triazin-2-yle, qui est non substitué ou substitué, et
R représente un atome d'hydrogène, un groupe alkyle en C₁ à C₅, halogénoalkyle en C₁ à C₅, alcoxy en C₁ à C₅, ou halogénoalcoxy en C₁ à C₅,
caractérisé en ce que l'on transforme un composé de formule (II),
H-NR-B (II)
avec une base M^{⊕}Base⁻, dans laquelle M^{⊕} représente l'équivalent d'un cation et Base⁻ l'équivalent anionique d'une base oxygénée,
pour donner le sel correspondant de formule (II-S),
M^{⊕ -}NR-B (II-S)
et en ce qu'on met à réagir le mélange réactionnel obtenu avec un carbonate de diaryle de formule (III),
(Ar-O)₂CO (III)
pour former le composé (IV),
formules (II), (II-S) et (III) dans lesquelles les symboles Ar, B et R sont définis comme dans la formule (IV).

2. Procédé selon la revendication 1, caractérisé en ce que, l'on met en oeuvre comme composé de formule (II) un composé de formule (IIa), 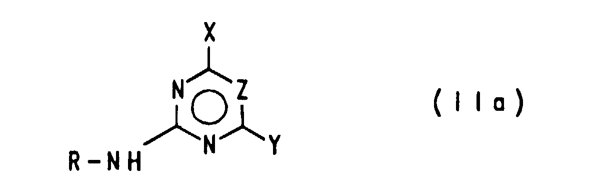 dans laquelle
R représente un atome d'hydrogène, un groupe alkyle en C₁ à C₅, halogénoalkyle en C₁ à C₅, alcoxy en C₁ à C₅ ou halogénoalcoxy en C₁ à C₅,
X, Y représentent indépendamment l'un de l'autre un atome d'hydrogène, d'halogène, un groupe alkyle en C₁ à C₆, alcoxy en C₁ à C₆, alkylthio en C₁ à C₆, mono- et di-(alkyle en C₁ à C₄)amino, dans lesquels chacun des six groupes cités précédemment est non substitué dans la partie alkyle ou est substitué par un ou plusieurs groupes choisis parmi un atome d'halogène, un groupe alcoxy en C₁ à C₄, halogénoalcoxy en C₁ à C₄, et alkylthio en C₁ à C₄, ou cycloalkyle en C₃ à C₆, alcényle en C₂ à C₆, alcynyle en C₂ à C₆, alcényloxy en C₂ à C₆ ou alcynyloxy en C₂ à C₆, et
Z représente CH ou N.

3. Procédé selon la revendication 1 ou 2, caractérisé en ce que, l'on met en oeuvre comme composé de formule (III) un composé de formule (IIIa), 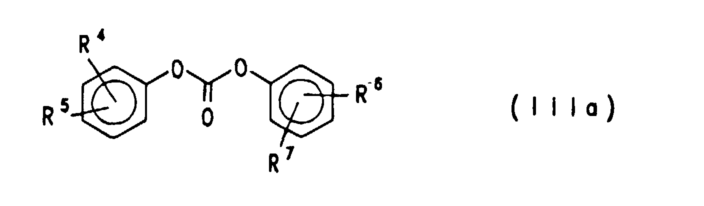 dans laquelle
R₄, R₅, R₆ et R₇ représentent indépendamment les uns des autres un atome d'hydrogène, d'halogène, un groupe alkyle en C₁ à C₄, halogénoalcoxy en C₁ à C₄, halogénoalkyle en C₁ à C₄, alcoxy en C₁ à C₄ ou CN.

4. Procédé selon une ou plusieurs des revendications 1 à 3, caractérisé en ce que, l'on utilise comme Base M^{⊕}Base⁻ des sels choisis parmi les alcoolates de métaux alcalins.

5. Procédé selon l'une des revendications 1 à 4, caractérisé en ce que, l'on utilise comme Base M^{⊕}Base⁻ des sels choisis parmi les alcoolates de métaux alcalins dans un solvant dipolaire aprotique.

6. Procédé selon la revendication 5, caractérisé en ce que, l'on utilise la base à des températures de -40°C jusqu'au point d'ébullition respectif du solvant.

7. Procédé selon l'une des revendications 1 à 6, caractérisé en ce que, l'on réalise la réaction donnant le carbamate (IV) à des températures de -40 à 180°C.

8. Procédé selon l'une des revendications 1 à 7, caractérisé en ce que, l'on met à réagir le mélange réactionnel obtenu, qui contient le composé de formule (IV), avec un sulfamide de formule (V) pour obtenir le composé de formule (I) ou un sel de celui-ci,
Ar-O-CO-NR-B (IV)
A-SO₂NH₂ (V)
A-SO₂-NH-CO-NR-B (I)
formules (IV), (V) et (I) dans lesquelles A représente un groupe organique, qui contient éventuellement des hétéroatomes, et les symboles Ar, A, B et R sont définis dans la formule (I) comme dans la formule (IV),
et on isole éventuellement ensuite le composé de formule (I) ou son sel.

9. Procédé pour la préparation d'un composé de formule (I) ou de ses sels,
A-SO₂-NH-CO-NR-B (I)
dans laquelle
A représente un groupe organique qui contient éventuellement des hétéroatomes,
B représente un groupe pyrimidin-2-yle ou 1,3,5-triazin-2-yle, qui est non substitué ou substitué, et
R représente un atome d'hydrogène, un groupe alkyle en C₁ à C₅, halogénoalkyle en C₁ à C₅, alcoxy en C₁ à C₅, ou halogénoalcoxy en C₁ à C₅,
caractérisé en ce que l'on transforme un composé de formule (II)
H-NR-B (II)
avec une base M^{⊕}Base⁻, dans laquelle M^{⊕} représente l'équivalent d'un cation et Base⁻ l'équivalent anionique d'une base oxygénée,
pour donner le sel correspondant de formule (II-S),
M^{⊕ -}NR-B (II-S)
et en ce qu'on met à réagir le mélange réactionnel obtenu avec un carbonate de diaryle de formule (III),
(Ar-O)₂CO (III)
dans laquelle
Ar représente un groupe aryle non substitué ou substitué, d'où il résulte le carbamate de formule (IV),
Ar-O-CO-NR-B (IV)
et en ce qu'on met à réagir le mélange réactionnel obtenu avec un sulfonamide de formule (V)
A-SO₂NH₂ (V)
et on isole éventuellement ensuite le composé de formule (I) ou un sel de celui-ci,
formules (II), (II-S), (IV) et (V) dans lesquelles les symboles A, B et R sont définis comme dans la formule (I).

10. Procédé selon la revendication 9, caractérisé en ce que, l'on réalise la réaction du carbamate (IV) avec le sulfonamide de formule (V) à des températures de -40 à 180°C.
